# EUROPÄISCHE PATENTSCHRIFT

(11) **EP 4 179 218 B1**
(45) Veröffentlichungstag und Bekanntmachung des Hinweises auf die Patenterteilung: **07.08.2024**
(21) Anmeldenummer: 21736590.7
(22) Anmeldetag: 24.06.2021
(51) Int. Cl.: F16B 5/01, F16B 37/04, F16B 37/12, F16B 11/00, F16B 13/14

(54) **MEHRTEILIG BUCHSENARTIGER EINKLEBEHALTER**
MULTI-PART SOCKET-LIKE CEMENT-IN RETAINER
DISPOSITIF DE RETENUE DANS DU CIMENT DU TYPE DOUILLE À MULTIPLES PARTIES

(30) Priorität: 07.07.2020 DE 102020117921
(43) Veröffentlichungstag der Anmeldung: 17.05.2023
(73) Patentinhaber: Böllhoff Verbindungstechnik GmbH, 33649 Bielefeld (DE)
(72) Erfinder: JODELEIT, Martin, 33617 Bielefeld (DE); DRÜKE, Franz, 32758 Detmold (DE); MATTHES, Jörg, 32051 Herford (DE); MEILWES, Peter, 33184 Altenbeken (DE)
(74) Vertreter: HWP Intellectual Property
(86) Internationale Anmeldenummer: PCT/EP2021/067416
(87) Internationale Veröffentlichungsnummer: WO 2022/008262

(56) Entgegenhaltungen:
- DE-A1- 102016 125 664
- US-A1- 2008 008 558
- US-A1- 2008 292 425
- US-B2- 9 702 394

## Beschreibung

### 1. Gebiet der Erfindung

Die vorliegende Erfindung betrifft einen mehrteilig buchsenartigen Einklebehalter, der in einer Bauteilöffnung klebend befestigbar ist. Der Einklebehalter lässt sich vor dem Einkleben in der Bauteilöffnung mit einem Funktionseinsatz ausstatten, so dass der Einklebehalter in Kombination mit dem passenden Funktionseinsatz in verschiedenen technischen Situationen anwendbar ist. Zudem offenbart vorliegende Erfindung ein Bauteil mit einer Bauteilöffnung, in der der oben genannte Einklebehalter mit Funktionseinsatz installiert ist. Des Weiteren offenbart vorliegende Erfindung ein Installationsverfahren für den Einklebehalter in der Bauteilöffnung sowie ein Herstellungsverfahren für den Einklebehalter gemäß oben beschriebener Konstruktion.

### 2. Hintergrund der Erfindung

Im Stand der Technik ist es ein generelles Problem, Befestigungsmöglichkeiten in Bauteilen vorzusehen, die aus einem wenig mechanisch belastbaren Material bestehen. Derartige Bauteile oder Platten werden auch als Leichtbauplatten bezeichnet. Sie bestehen nach einer Materialalternative aus einem Wabenmaterial oder werden allgemein als Wabenplatte bezeichnet. Gemäß einer anderen Konstruktionsalternative handelt es sich bei diesen Leichtbauplatten um Sandwichplatten oder Platten aus Schaummaterial.

Leichtbauplatten liefern aufgrund des Bauteilmaterials keine ausreichenden Widerstandskräfte, um beispielsweise einen Gewindebolzen direkt mit der Leichtbauplatte zu verbinden. Daher sind in derartigen Leichtbauplatten häufig Löcher vorgesehen, in welche Bauteileinsätze eingeklebt sind. Derartige Bauteileinsätze sind beispielsweise Gewindeeinsätze aus Metall, welche in der Bauteilöffnung, vorzugsweise eine Sacklochbohrung, eingeklebt sind. Eine derartige Konstruktion ist in US 4,729,705 beschrieben. Um den Gewindeeinsatz in der Sacklochbohrung der Leichtbauplatte zu befestigen, wird die Sacklochbohrung zum Teil mit Klebstoff gefüllt. Durch das Einsetzen des Gewindeeinsatzes in die Sacklochbohrung wird der Klebstoff in den Spalt zwischen Innenwand der Sacklochbohrung und äußerer Mantelfläche des Gewindeeinsatzes verdrängt. Dieses Verdrängungsprinzip hat den Nachteil, dass nicht alle zu benetzenden Oberflächen des Gewindeeinsatzes sowie der Sacklochbohrung durch den Klebstoff benetzt werden, um eine verlässliche Befestigung des Gewindeeinsatzes in der Sacklochbohrung zu erzielen. Ein weiterer Nachteil besteht darin, dass verdrängter Klebstoff aus der Sacklochbohrung herausgedrückt wird und auf diese Weise das Erscheinungsbild des eingeklebten Gewindeeinsatzes in der Sacklochbohrung beeinträchtigt.

In DE 10 2011 009 334 A1 ist ein Beschlagsystem mit zwei Beschlagteilen beschrieben, die ebenfalls in Leichtbauplatten installierbar sind. Derartige Beschlagsysteme werden in der Möbelindustrie eingesetzt, um passende Verwendungsmöglichkeiten für derartige Leichtbauplatten bereitzustellen. Die beschriebenen Beschlagteile sind an ihren Mantelflächen mit einem Klebstoff ausgestattet, der mittels Wärme aktivierbar ist. Zu diesem Zweck wird das jeweilige Beschlagteil in eine Sacklochbohrung oder eine andere passende Öffnung eingesetzt und dort mit hoher Geschwindigkeit gedreht. Durch die Reibung zwischen der Innenwand der Öffnung und der Außenseite des Beschlagteils mit Klebstoff entsteht Wärme, welche wiederum den Klebstoff auf der Außenseite des Beschlagteils aktiviert.

Eine verlässliche Verbindung zwischen Beschlagteil und Innenwand der Bauteilöffnung ist aber nur dann gegeben, wenn der Innendurchmesser der Bauteilöffnung genau an den Außendurchmesser des Beschlagteils angepasst ist. Denn andernfalls entsteht keine Wärme, welche den Klebstoff auf der Außenseite des Beschlagteils aktiviert. Ein weiterer Nachteil besteht darin, dass die Klebstoffmenge zur Befestigung des Beschlagteils in der Bauteilöffnung nicht ausreichend ist, sofern der Spalt zwischen Beschlagteil und Innenwand der Bauteilöffnung zu groß ist. In diesem Fall wird zwar durch Reibung der Klebstoff aktiviert, die vorhandene Klebstoffmenge reicht jedoch nicht aus, um den vorhandenen Spalt zwischen Beschlagteil und Bauteilöffnung zu füllen.

Zudem beschreibt dieses Dokument lediglich eine angepasste Verbindung zwischen den beiden miteinander zu verbindenden Beschlagteilen. Diese Verbindung beschränkt sich auf eine kraftschlüssige und/oder formschlüssige Verbindung, die einer rastenden Steckverbindung ähnlich ist. Daraus ergibt sich ähnlich wie bei dem oben beschriebenen Gewindeeinsatz die fehlende Flexibilität im Hinblick auf Verbindungsmöglichkeiten dieser Beschlagteile. Denn die Verbindung ist jeweils beschränkt auf die innere Konstruktion, die das jeweilige Beschlagteil zur Verbindung bereitstellt.

US 4,812,193 beschreibt ebenfalls einen einzuklebenden Gewindeeinsatz für beispielsweise eine Wabenplatte. Dieser Gewindeeinsatz wird in einer Bauteilöffnung platziert, um nachfolgend in den Spalt zwischen Gewindeeinsatz und innerer Wand der Bauteilöffnung einen Klebstoff einzuspritzen. Dieser Klebstoff umströmt den Gewindeeinsatz und füllt den Spalt zwischen Gewindeeinsatz und Innenwand der Bauteilöffnung aus, um den Gewindeeinsatz verlässlich in der Bauteilöffnung zu befestigen. Um den Klebstoff für eine vorteilhafte Benetzung der Außenwand des Gewindeeinsatzes zu leiten, sind mehrere scheibenartige Radialstege vorgesehen. Diese Radialstege verlaufen in umfänglicher Richtung, sind gleichmäßig voneinander beabstandet und bilden Strömungsvertiefungen, in welche der Klebstoff hineinströmen kann. In axialer Richtung benachbart sind die Radialstege in einem begrenzten Abschnitt abgeflacht, um Überströmbereiche für den Klebstoff zwischen den benachbarten Vertiefungen zu erzeugen.

Von Nachteil ist bei dieser klebenden Befestigung des Gewindeeinsatzes ebenfalls die Sicherstellung, dass die Oberfläche des Gewindeeinsatzes ausreichend mit Klebstoff benetzt wird, um eine verlässliche Klebeverbindung zum Bauteil zu erzielen. Diese Benetzung wird nachteilig durch nicht erkennbare Lufteinschlüsse behindert.

Ein Gewindeeisatz zum Aufnehmen eines mit einem Gewinde versehenen Befestigers in einem Verbundpanel ist in US 2008/292425 A1 beschrieben. Der Gewindeeinsatz zur Verwendung in einer Verbund- und/oder Wabenmatrix hat ein Mutterelement, das in einem Kunststoffgehäuse angeordnet ist. Das Gewinde des Mutterelements hat einen freilaufenden Gewindeabschnitt und einen Gewindesicherungsabschnitt. Wenn ein Befestigungselement in das Mutternelement eingebaut wird, greift eine Oberfläche auf dem Mutternelement in eine gegenüberliegende Oberfläche auf dem Kunststoffgehäuse ein, wodurch die Mutterbelastung von einer Zugbelastung in eine Druckbelastung umgekehrt wird. Eine thermoplastische Ausführungsform des Mutterelements kann hergestellt werden, um die Spezifikationen von Flugzeugherstellern zu erfüllen, die für den SL2334-Einsatz erforderlich sind, einschließlich Gewindewiederverwendbarkeit, Zugbelastung (Auszugsbelastung), Scherung und Austauschbarkeit mit der derzeit verwendeten Metallkomponente.

In Bezug auf den oben beschriebenen Stand der Technik ist es daher die Aufgabe vorliegender Erfindung, eine alternative Konstruktion für einen Einklebehalter bereitzustellen, der in einer Bauteilöffnung klebend befestigbar ist und mit dem sich eine verlässliche Verbindung zu einem Funktionsteil, wie beispielsweise einem Gewindebolzen oder einer ähnlichen Komponente, herstellen lässt.

### 3. Zusammenfassung der Erfindung

Die obige Aufgabe wird gelöst durch einen mehrteilig buchsenartigen Einklebehalter gemäß dem unabhängigen Patentanspruch 1, ein Bauteil mit einer Bauteilöffnung gemäß dem unabhängigen Patentanspruch 12, in der ein derartiger Einklebehalter befestigt ist, ein Installationsverfahren des Einklebehalters in dem Bauteil gemäß dem unabhängigen Patentanspruch 13 sowie ein Herstellungsverfahren des Einklebehalters gemäß dem unabhängigen Patentanspruch 15. Vorteilhafte Ausgestaltungen und Weiterentwicklungen vorliegender Erfindung gehen aus der folgenden Beschreibung, den begleitenden Zeichnungen und den anhängenden Patentansprüchen, welche den Schutzumfang definieren, hervor.

Vorliegende Erfindung offenbart einen mehrteilig buchsenartigen Einklebehalter, der in einer Bauteilöffnung klebend befestigbar und aufgrund einer Axialteilung, insbesondere eine Teilung in Richtung einer Längsachse des Einklebehalters, zumindest zweiteilig und dadurch in Radialrichtung öffnend ausgebildet ist, sodass nur vor einem Einkleben des Einklebehalters in der Bauteilöffnung ein Funktionseinsatz in dem Einklebehalter anordenbar und nach dem Einkleben des Einklebehalters in der Bauteilöffnung der Funktionseinsatz in dem Einklebehalter untrennbar gehalten ist.

Der erfindungsgemäße Einklebehalter ist darauf gerichtet, Bauteile aus weniger mechanisch tragfähigen Materialien, wie beispielsweise Leichtbauplatten, Wabenplatten oder Schaum-/ Sandwichplatten, mit einer belastbaren Verbindungsmöglichkeit auszustatten. Zu diesem Zweck wird der Einklebehalter in einer im Bauteil vorgesehenen Öffnung, vorzugsweise eine Sacklochbohrung, eingeklebt. Der Einklebehalter bildet nach seiner klebenden Befestigung in dem Bauteil eine universelle Basis, für ein vor dem Einkleben eingesetztes Funktionselement oder Befestigungselement, welches in dem Einklebehalter nach dem Einkleben untrennbar gehalten ist. Somit stellt der Einklebehalter einerseits eine verlässliche und mechanisch belastbare Befestigung eines Funktionseinsatzes, wie beispielsweise das Befestigungselement oder eine Verbindungskomponente, dar. Andererseits ist der Einklebehalter vor dem Einkleben mit einem Funktionseinsatz entsprechend den Erfordernissen der herzustellenden Bauteilverbindung kombinierbar. Das bedeutet, dass eine Auswahl von Funktionseinsätzen zur Verfügung steht, die in gleicher Weise in dem Einklebehalter vor dem Einkleben angeordnet und nach dem Einkleben in der Bauteilöffnung untrennbar gehalten werden können. Zu diesem Zweck ist der Einklebehalter schalenartig aufgebaut. Diese Schale lässt sich zum Aufnehmen des Funktionseinsatzes zerstörungsfrei öffnen und nach dem Einsetzen des Funktionseinsatzes wieder schließen. Damit stellt der erfindungsgemäße Einklebehalter in Kombination mit unterschiedlichen Funktionseinsätzen bzw. Befestigung- oder Verbindungeinsätzen einen Baukasten bereit, sodass sich der Einklebehalter in Kombination mit seinem Funktionseinsatz an die jeweils erforderliche Verbindungssituation des Bauteils anpassen lässt.

Gemäß einer bevorzugten Ausgestaltung vorliegender Erfindung weist der Einklebehalter einen hohlzylindrischen Aufnahmekörper mit einer umlaufenden Mantelfläche, einer geschlossenen axialen Stirnseite und einer offenen axialen Stirnseite auf, wobei angrenzend an die offene Stirnseite ein umfänglich umlaufender Befestigungskragen angeordnet ist.

Der Einklebehalter wird vorzugsweise durch einen topfartigen oder hohlzylindrischen Aufnahmekörper für den Funktionseinsatz gebildet. Dieser topfartige Aufnahmekörper ist vorzugsweise an einer Seite geschlossen, um einen verlässlichen Halt des Funktionseinsatzes zu gewährleisten und zudem eine Klebefläche des Einklebehalters in der Bauteilöffnung bereitzustellen. Gegenüber der geschlossenen axialen Stirnseite ist eine Funktionsöffnung vorgesehen, um den Funktionseinsatz, der innerhalb des Aufnahmekörpers untrennbar gehalten ist, nutzen zu können oder zugänglich zu machen. Diese Funktionsöffnung wird durch die offene axiale Stirnseite realisiert, welche der geschlossenen axialen Stirnseite zum klebenden Befestigen des Einklebehalters in der Bauteilöffnung gegenüberliegt.

Während einerseits bevorzugt eine axiale Stirnseite des Aufnahmekörpers geschlossen ausgebildet ist, umgibt ein umfänglich umlaufender Befestigungskragen die Funktionsöffnung an der offenen Stirnseite des Aufnahmekörpers. Vorzugsweise ist der Befestigungskragen am offenen axialen Ende des Aufnahmekörpers angeordnet. Er erstreckt sich radial in Bezug auf die Mantelfläche des Aufnahmekörpers und fluchtet bevorzugt mit der offenen Stirnseite des Aufnahmekörpers. Auf diese Weise wird die bevorzugte Möglichkeit geschaffen, dass der Befestigungskragen auf einer Bauteiloberfläche aufliegt, während sich der restliche Aufnahmekörper in die Bauteilöffnung hinein erstreckt. Dies eröffnet die bevorzugte Möglichkeit, dass der Einklebehalter nicht nur über die geschlossene axiale Stirnseite des Aufnahmekörpers in der Bauteilöffnung klebend befestigt werden kann. Denn auch die an die Bauteiloberfläche angrenzende Unterseite des Befestigungskragens stellt eine bevorzugte Klebefläche dar, sodass mithilfe eines Klebstoffs eine stoffschlüssige Verbindung zwischen der Unterseite des Befestigungskragens und der benachbart dazu angeordneten Bauteiloberfläche ein verlässlicher Halt des Einklebehalters in der Bauteilöffnung erzielt werden kann.

Gemäß einer weiteren bevorzugten Ausführungsform vorliegender Erfindung ist der Aufnahmekörper aufgrund mindestens eines Axialschnitts in Längsrichtung des Einklebehalters in mindestens zwei relativ zueinander bewegbare, insbesondere aufklappbar versetzbare, Teile geteilt, die vollständig oder teilweise voneinander lösbar sind.

Gemäß einer erfindungsgemäß bevorzugten Konstruktion des Aufnahmekörpers, stellt dieser keinen in sich geschlossenen topfartigen Körper bereit. Vielmehr ist der topfartige Aufnahmekörper in seiner Längsrichtung geteilt ausgebildet. Diese Teilung in mindestens zwei Teile, vorzugsweise zwei Hälften, gewährleistet ein Öffnen des Aufnahmekörpers zur Aufnahme des Funktionseinsatzes und ein nachfolgendes Schließen, sodass der Funktionseinsatz untrennbar innerhalb des Aufnahmekörpers und somit des Einklebehalters gehalten ist.

Entsprechend stellt der Aufnahmekörper vorzugsweise eine Schalenkonstruktion bereit, die sich funktionell öffnen und schließen lässt. Diese bevorzugte mehrteilige Schalenkonstruktion stellt mit ihrer Außenfläche, vorzugsweise die umlaufende Mantelfläche des Aufnahmekörpers, eine Befestigungsfläche und/oder Befestigungskonstruktion zum klebenden Befestigen des Aufnahmekörpers in der Bauteilöffnung bereit. Die Innenseite der mehrteiligen Schalenkonstruktion dient dem verlässlichen Halten des Funktionseinsatzes, nachdem die Schalenkonstruktion wieder geschlossen worden ist. Zu diesem Zweck weist die Schalenkonstruktion an ihrer Innenseite bevorzugt eine Formgestaltung auf, die einen Formschluss mit dem aufzunehmenden und zu haltenden Funktionseinsatz gewährleistet, nachdem der Aufnahmekörper wieder geschlossen worden ist.

Gemäß unterschiedlicher bevorzugter Ausgestaltungen des mehrteiligen Aufnahmekörpers unterteilt der mindestens eine Axialschnitt den Aufnahmekörper in zwei oder mehr relativ zueinander bewegbare Teile. Diese zueinander bewegbaren Teile sind gemäß einer bevorzugten Ausgestaltung vollständig voneinander getrennt ausgebildet. Gemäß einer weiteren bevorzugten Ausgestaltung sind die mindestens zwei Teile an ausgewählten Verbindungsstellen derart bewegbar miteinander verbunden, dass die mehreren Teile des Aufnahmekörpers so weit geöffnet werden können, dass der Funktionseinsatz im Inneren des Aufnahmekörpers anordenbar und nachfolgend der Aufnahmekörper wieder verschließbar ist.

Gemäß einer bevorzugten Ausgestaltung vorliegender Erfindung besteht der Aufnahmekörper aus zwei Halbschalen, die sich voneinander lösen und wieder verbinden lassen.

Gemäß einer bevorzugten Ausgestaltung vorliegender Erfindung sind entlang des Axialschnitts Verbindungsflächen der mehreren Teile des Aufnahmekörpers vorgesehen, die einander gegenüber liegen und Positionierhilfen und/oder Verbindungshilfen der Teile aufweisen. Die Verbindungsflächen der mehreren Teile des Aufnahmekörpers weisen beispielsweise an einer Hälfte des Aufnahmekörpers Stifte auf, die in entsprechend angeordnete Vertiefungen der anderen Hälfte des Aufnahmekörpers eingreifen. Diese formschlüssige Verbindung kann zusätzlich bevorzugt mit einer Rastverbindung kombiniert sein.

Gemäß einer weiteren Alternative sind die mehreren Teile des Aufnahmekörpers zumindest einseitig mit einem Filmscharnier miteinander verbunden. Ein derartiges Filmscharnier ermöglicht die Bewegbarkeit, d. h. ein Aufklappen des Aufnahmekörpers um das Filmscharnier, sodass der Funktionseinsatz in den Aufnahmekörper einsetzbar ist.

Gemäß einer weiteren bevorzugten Ausgestaltung vorliegender Erfindung sind die aneinander angrenzenden Teile des Aufnahmekörpers über das oben genannte Filmscharnier und/oder eine Schnappverbindung und/oder eine Steckverbindung bewegbar miteinander verbunden.

Gemäß einer weiteren bevorzugten Ausgestaltung vorliegender Erfindung weist die Mantelfläche des Einklebehalters eine strömungsleitende Außenseite angepasst an eine Leitung eines strömenden Klebstoffs sowie eine Funktionsinnenseite auf, mit der der Funktionseinsatz formschlüssig in dem geschlossenen Aufnahmekörper haltbar ist.

Die Funktionsinnenseite des Aufnahmekörpers ist vorzugsweise gestaltet, um eine formschlüssige Verbindung mit dem aufzunehmenden und zu haltenden Funktionseinsatz auszubilden. Zu diesem Zweck werden unter anderem Nut-Feder-Verbindungen eingesetzt, wobei entweder die Funktionsinnenseite als Nut eine umlaufende Vertiefung aufweist, in welche ein radial vom Funktionseinsatz abstehender Kragen eingreift. In gleicher Weise ist es ebenfalls bevorzugt, dass ein derartiger Kragen an der Funktionsinnenseite des Aufnahmekörpers vorgesehen ist, der in eine entsprechende Nut am Funktionseinsatz eingreift.

Erstreckt sich ein derartiger Kragen senkrecht zur Längsachse des Aufnahmekörpers, wird durch die Nut-Feder-Verbindung der Funktionseinsatz in axialer Richtung innerhalb des Aufnahmekörpers fest angeordnet. Gleichzeitig erlaubt eine derartige Verbindung auch, dass der Funktionseinsatz senkrecht zur Längsachse des Aufnahmekörpers fest oder schwimmend angeordnet ist. Die schwimmende Anordnung eröffnet die Möglichkeit, dass ein gewisser Toleranzausgleich bei der Nutzung des Funktionseinsatzes bereitgestellt werden kann. Dies spielt beispielsweise dann eine Rolle, wenn der Funktionseinsatz einen Gewindeeinsatz oder eine Kupplungsaufnahme darstellt, in die von der Ausrichtung passend ein Gewindebolzen eingeschraubt oder ein Kupplungsbolzen eingesteckt werden muss.

Ebenfalls bevorzugt sind an der strömungsleitenden Außenseite vorstehende Stege mit unterschiedlicher Ausrichtung angeordnet. Diese Stege bilden Begrenzungen für vertieft angeordnete Flächen oder Kanäle, in welchen ein den Aufnahmekörper umströmender Klebstoff aufgenommen und geleitet wird. Zudem ist es bevorzugt, derartige Stege mit Unterbrechungen vorzusehen, sodass ein Überströmen der Stege gewährleistet ist.

Gemäß einer weiteren bevorzugten Ausgestaltung vorliegender Erfindung weist die Funktionsinnenseite des Aufnahmekörpers eine umlaufende Haltenut auf, die in einer Breite und in einer Tiefe an einen umlaufenden Kragen des Funktionseinsatzes angepasst ist, um diesen fest oder schwimmend innerhalb des Aufnahmehohlraums zu halten (siehe oben).

Gemäß einer weiteren bevorzugten Ausgestaltung vorliegender Erfindung weist der Befestigungskragen des Einklebehalters eine Mehrzahl von Durchstrahlabschnitten auf, in denen eine Stärke des Befestigungskragens in Längsrichtung des Einklebehalters reduziert ist, um den Befestigungskragen in den Durchstrahlabschnitten mit Licht durchstrahlbar vorzusehen.

Gemäß einer bevorzugten Ausgestaltung vorliegender Erfindung wird der Einklebehalter zwischen der Bauteiloberfläche und der angrenzenden Unterseite des Befestigungskragens mithilfe eines Klebstoffs befestigt. Um diesen Klebstoff zu aktivieren und/oder auszuhärten, ist es erforderlich, den Klebstoff mit Licht einer bestimmten Wellenlänge zu bestrahlen. Da jedoch aus ästhetischen Gründen vorzugsweise der Befestigungskragen die Klebeverbindung zur Bauteiloberfläche abdeckt, wäre die Klebstoffmenge unterhalb des Befestigungskragens für eine Lichteinstrahlung nicht erreichbar. Daher sind bevorzugt die Durchstrahlabschnitte vorgesehen. Diese sind aufgrund ihrer Dicke mit Licht durchstrahlbar, sodass die nötige Lichtintensität die Durchstrahlbereiche durchdringt, auf dem Klebstoff auftrifft und diesen aktiviert und/oder aushärtet.

Gemäß einer weiteren bevorzugten Ausgestaltung des erfindungsgemäßen Einklebehalters ist innerhalb des Aufnahmekörpers an der geschlossenen axialen Stirnseite ein Blockiersteg vorgesehen, der in einer Blockiernut des Funktionseinsatzes aufnehmbar ist, sodass eine Relativdrehung zwischen dem Funktionseinsatz und dem Aufnahmekörper verhindert ist.

Gemäß einer weiteren bevorzugten Ausführungsform des Einklebehalters ist der Funktionseinsatz eine einseitig geschlossene Hülse mit umlaufendem Kragen und einem Innengewinde oder einer planen Innenwand, eine Federklammer für einen Schnellverschluss, eine Kugelpfanne mit einem umlaufenden Kragen oder ein vorstehender Gewindezapfen oder ein Kugelbolzen.

Gemäß einer weiteren bevorzugten Ausführungsform des erfindungsgemäßen Einklebehalters weist der hohlzylindrische Aufnahmekörper an einer umlaufenden Innenwand ein Innengewinde auf, in dem als Funktionseinsatz ein Drahtgewindeeinsatz angeordnet ist.

In gleicher Weise ist es ebenfalls bevorzugt, innerhalb des Aufnahmekörpers einen hohlzylindrischen Funktionseinsatz anzuordnen, der an seiner Innenseite ein Innengewinde mit einem darin angeordneten Drahtgewindeeinsatz aufweist. Im Unterschied zu der oben beschriebenen Alternative eröffnet der Funktionseinsatz mit Innengewinde und Drahtgewindeeinsatz eine schwimmende Lagerung im Aufnahmekörper, um eventuelle Toleranzen beim Einschrauben eines Gewindebolzens in das Innengewinde mit Drahtgewindeeinsatz ausgleichen zu können.

Im Hinblick auf die oben beschriebenen bevorzugten Ausgestaltungsalternativen vorliegender Erfindung bestehen vorzugsweise der Einklebehalter und/oder der Funktionseinsatz aus einem thermoplastischen Kunststoff mit einer Dauergebrauchstemperatur von mindestens 130°C. Diese bevorzugte Materialwahl gewährleistet den bevorzugten Einsatz vorliegender Erfindung in der Luftfahrttechnik.

Alternativ zur obigen Materialwahl ist es ebenfalls bevorzugt, gängige Kunststoffe von Haushaltsgegenständen, der Automobilindustrie oder anderer Branchen zu nutzen. Entsprechend kommen vorzugsweise Polyamide und ähnliche Kunststoffe zum Einsatz.

Vorliegende Erfindung offenbart zudem ein Bauteil mit einer Bauteilöffnung, vorzugsweise eine Sacklochbohrung, in der ein Einklebehalter gemäß einer der oben beschriebenen bevorzugten Ausgestaltungen eingeklebt ist.

Vorliegende Erfindung offenbart zudem ein Installationsverfahren eines Einklebehalters gemäß einer der oben beschriebenen bevorzugten Ausgestaltungen in einem Bauteil. Das Installationsverfahren weist die folgenden Schritte auf: Bereitstellen eines Bauteils mit einer Sacklochbohrung, in die der Einklebehalter aufnehmbar ist, Anordnen eines Funktionseinsatzes in dem Einklebehalter, so dass der Funktionseinsatz unlösbar in dem Einklebehalter gehalten ist, und danach Einkleben des Einklebehalters in der Sacklochbohrung.

In einer bevorzugten Ausgestaltung des Installationsverfahrens erfolgt in weiteren Schritten ein Applizieren einer ersten Klebstoffmenge an einem Boden der Sacklochbohrung, ein Einsetzen des Einklebehalters mit Funktionseinsatz in die Sacklochbohrung mit der ersten Klebstoffmenge und ein Aushärten des Klebstoffs.

Zudem ist es bevorzugt, eine zweite Klebstoffmenge zwischen dem umlaufenden stirnseitigen Axialkragen des Einklebehalters und dem Bauteil zu applizieren und vorzugsweise dort auszuhärten.

Die unterschiedlichen bevorzugten Ausgestaltungen des Installationsverfahrens eröffnen die Möglichkeit, den Einklebehalter auf unterschiedliche Art und Weise in der Bauteilöffnung klebend zu befestigen. Gemäß einer Alternative wird die Bauteilöffnung zum Teil mit Klebstoffs gefüllt und durch das Einsetzen des Einklebehalters der Klebstoff in den Spalt zwischen der Innenwand der Bauteilöffnung und der Außenseite des Einklebehalters verdrängt. Das dortige Aushärten des Klebstoffes befestigt den Einklebehalter in der Bauteilöffnung.

Gemäß einer bevorzugten Ausgestaltung vorliegender Erfindung lässt sich der Einklebehalter auch durch das Aufbringen nur einer Klebstoffmenge am Boden der Bauteilöffnung befestigen. Auf diese Klebstoffmenge trifft die geschlossene axiale Stirnseite des Einklebehalters auf, während der Einklebehalter in die Bauteilöffnung eingesetzt wird. Auf diese Weise wird die Klebstoffmenge in den Spalt zwischen Innenwand der Bauteilöffnung und Außenseite des Einklebehalters verdrängt. Vorzugsweise ist die Klebstoffmenge ausreichend klein gewählt, sodass nur die geschlossene axiale Stirnseite und die ihr gegenüberliegende Oberfläche der Bauteilöffnung sowie die Außenwand des Einklebehalters bis maximal zur halben axialen Länge des Einklebehalter mit dem Klebstoff benetzt wird. Auf diese Weise wird vermieden, dass zu viel Klebstoff aus der Bauteilöffnung während des Einsetzens des Einklebehalters verdrängt wird und dadurch das ästhetische Erscheinungsbild des Bauteils stört.

Gemäß einer weiteren bevorzugten Ausgestaltung wird der Einklebehalter gezielt mit dem Applizieren von mindestens zwei Klebstoffmengen in der Bauteilöffnung befestigt. Dazu wird eine erste Klebstoffmenge am Boden der Sacklochbohrung aufgebracht, sodass eine Klebstoffverbindung zwischen der geschlossenen axialen Stirnseite des Einklebehalters und dem Grund der Sacklochbohrung herstellbar ist. Eine zweite Klebstoffmenge wird vorzugsweise unterhalb des Befestigungskragens aufgebracht. Auf diese Weise wird eine zweite Klebeverbindung zwischen der Unterseite des Befestigungskragens und der ihr gegenüberliegenden Bauteiloberfläche hergestellt.

Gemäß einer weiteren bevorzugten Ausgestaltung des erfindungsgemäßen Installationsverfahrens wird der Einklebehalter mit zwei verschiedenen Klebstoffen befestigt, die unterschiedlichen Aushärteprinzipien gehorchen.

Am Boden der Sacklochbohrung wird bevorzugt ein Klebstoff aufgebracht, der ohne spezielle Aktivierung über die Zeit aushärtet. Es ist zudem bevorzugt, einen Klebstoff aufzubringen, der zunächst mit dem Einstrahlen von Licht aktiviert wird, um dann in der nachfolgenden Zeit auszuhärten. Zu diesem Zweck ist es beispielsweise denkbar, dass der Klebstoff in der Sacklochbohrung appliziert wird, dann Licht zum Aktivieren eingestrahlt wird und danach erst der Einklebehalter in die Sacklochbohrung mit Klebstoff eingesetzt wird. Gemäß einer weiteren bevorzugten Alternative des Installationsverfahrens härtet der applizierte Klebstoff in der Sacklochbohrung durch die Einstrahlung von Wärme aus.

Alternativ ist es bevorzugt, unterhalb des Befestigungskragens einen lichtaktivierbaren und/oder einen lichtaushärtenden Klebstoff vorzusehen. Denn mithilfe der bevorzugten oben beschriebenen Durchstrahlbereiche im Befestigungskragen ist es möglich, die Klebstoffmenge zwischen der Unterseite des Befestigungskragens und der Bauteiloberfläche mittels Lichteinstrahlung zu aktivieren. In gleicher Weise ist es bevorzugt, zwischen der Unterseite des Befestigungskragens und der Oberfläche des Bauteils einen durch Wärme aushärtenden Klebstoff oder einen über Zeitablauf aushärtenden Klebstoff vorzusehen. Sofern ein wärmeaushärtender Klebstoff verwendet wird, ist es erforderlich, dass die hergestellte Klebestelle ausreichend erwärmt wird, um den Aushärteprozess zu unterstützen.

Zudem umfasst vorliegende Erfindung ein Herstellungsverfahren für den Einklebehalter gemäß der oben beschriebenen bevorzugten Ausgestaltungen. Das Herstellungsverfahren weist die folgenden Schritte auf: Bereitstellen einer ersten Spritzgussform, die komplementär zu der Form des Einklebehalters ausgebildet ist, Spritzgießen des Einklebehalters in der ersten Spritzgussform und des Funktionseinsatzes in der zweiten Spritzgussform und Entformen des Einklebehalters.

Gemäß einer weiteren bevorzugten Ausgestaltung des Herstellungsverfahrens sind die zusätzlichen Schritte vorgesehen: Bereitstellen einer zweiten Spritzgussform, die komplementär zu der Form des Funktionseinsatzes ausgebildet ist, Spritzgießen des Funktionseinsatzes in der zweiten Spritzgussform und Entformen des Funktionseinsatzes. Alternativ dazu ist es ebenfalls bevorzugt, einen vorgefertigten Funktionseinsatz bereitzustellen.

Vorzugsweise werden der Einklebehalter und/oder der Funktionseinsatz aus einem thermoplastischen Kunststoff mit einer Dauergebrauchstemperatur von mindestens 130°C hergestellt.

### 4. Kurzbeschreibung der begleitenden Zeichnungen

Die bevorzugten Ausführungsformen vorliegender Erfindung werden unter Bezugnahme auf die begleitenden Zeichnungen näher erläutert. Es zeigen:
- Figur 1: eine schematische Darstellung eines Bauteils mit einer Bohrung, in die eine bevorzugte Ausgestaltung des Einklebehalters eingesetzt werden soll,
- Figur 2: ein schematisch dargestelltes Bauteil mit einer Bauteilöffnung, in der eine bevorzugte Ausgestaltung des Einklebehalters mit einem Funktionseinsatz befestigt ist,
- Figur 3: eine perspektivische Seitenansicht einer bevorzugten Ausgestaltung des Einklebehalters,
- Figur 4: eine Explosionsdarstellung des Einklebehalters gemäß Figur 3 mit einer bevorzugten Ausführungsform eines darin angeordneten Funktionseinsatzes,
- Figur 5A bis D: verschiedene bevorzugte Ausgestaltungen des Funktionseinsatzes
- Figur 6: eine weitere bevorzugte Ausgestaltung des Einklebehalters
- Figur 7: eine weitere bevorzugte Ausgestaltung des Einklebehalters mit einem Gewindebolzen als Funktionseinsatz,
- Figur 8: eine weitere bevorzugte Ausführungsform des Einklebehalters mit einem Kugelbolzen als Funktionseinsatz,
- Figur 9: eine weitere bevorzugte Ausgestaltung des Einklebehalters in einer Explosionsdarstellung mit einem Innengewinde und einem darin anzuordnenden Drahtgewindeeinsatz,
- Figur 10: eine schematische Schnittdarstellung eines in die Bauteilöffnung eingeklebten Einklebehalters,
- Figur 11: ein Flussdiagramm einer bevorzugten Ausführungsform des erfindungsgemäßen Installationsverfahrens.

### 5. Detaillierte Beschreibung der bevorzugten Ausführungsformen

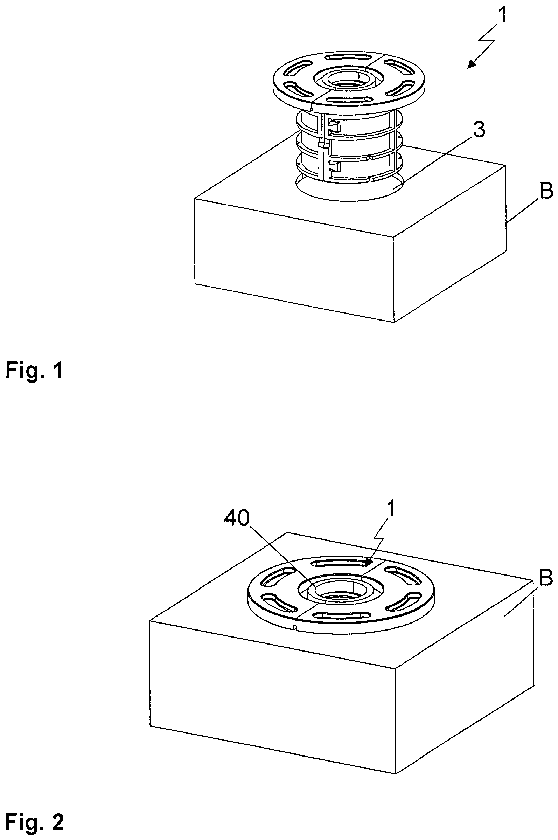

Figur 1 zeigt eine schematische Darstellung eines Bauteils B mit einer Bauteilöffnung 3, bevor ein mehrteilig buchsenartiger Einklebehalter 1 darin eingeklebt wird. Der Einklebehalter 1, auf den unten näher eingegangen wird, eignet sich bevorzugt für Bauteile B geringer mechanischer Eigenstabilität im Vergleich zu Metall oder Holz, wie beispielsweise Leichtbauplatten, Sandwichplatten, Wabenplatten, Schaumplatten und Kombinationen davon. Nichtsdestotrotz ist es ebenfalls bevorzugt, den Einklebehalter 1 in Bauteilen B beliebiger Materialien zu befestigen.

Der Einklebehalter 1 ist vorzugsweise als Spritzgussteil aus Kunststoff hergestellt. Alternativ ist es bevorzugt, den Einklebehalter 1 mit bekannten additiven Herstellungsverfahren herzustellen. Dies eröffnet ebenfalls die Möglichkeit, den Einklebehalter 1 aus Metall bereitzustellen, beispielsweise mittels Lasersintern.

Gemäß einer bevorzugten Ausgestaltung vorliegender Erfindung besteht der Einklebehalter 1 und bevorzugt auch ein darin gehaltener Funktionseinsatz 40 aus einem thermoplastischen Kunststoff mit einer Dauergebrauchstemperatur von mindestens 130 °C, insbesondere ein Hochleistungskunststoff. Hochleistungskunststoffe werden vorzugsweise in der Luftfahrtindustrie eingesetzt.

Der Begriff Hochleistungskunststoff oder Hochleistungsthermoplast basiert auf einer anwendungsbezogenen, ingenieurstechnischen Einteilung der thermoplastischen Kunststoffe, die zwischen Standardkunststoffen, technischen Kunststoffen und die Hochleistungskunststoffen unterscheidet. Wie der Name bereits andeutet erfüllen Hochleistungskunststoffe höhere Ansprüche als die Standard- und die technischen Kunststoffe. Insbesondere weisen sie bessere mechanische Eigenschaften, eine höhere Chemikalien- und/oder eine höhere Wärmebeständigkeit auf. Damit stehen sie insbesondere im Gegensatz zu den technischen Kunststoffen, die ein breites Funktionsspektrum abdecken.

Alle Hochleistungskunststoffe enthalten aromatische Strukturen. Da die aromatische Kohlenstoff-Wasserstoff-Bindung deutlich stabiler ist als die aliphatische Kohlenstoff-Wasserstoff-Bindung, wird aufgrund der Oxidationsbeständigkeit die bei einer thermischen Zersetzung oder Feuer auftretende Radikalbildung erschwert. Weiterhin ist die Kettensteifheit aromatischer Polymere größer als die von aliphatischen Polymeren, was die Glasübergangstemperatur bzw. im Fall von kristallinen Polymeren den Kristallitschmelzpunkt steigert und die Löslichkeit verringert. Aromatische Strukturen vereinen daher die beiden wichtigsten Merkmale für die Widerstandsfähigkeit gegen hohe Temperaturen. Die thermische Stabilität ist daher eine zentrale Eigenschaft der Hochleistungskunststoffe.

Ausgehend von den Eigenschaften der Standardkunststoffe können mechanische und thermische Verbesserungen bereits durch die Zugabe von Verstärkungsstoffen wie Glas- und Kohlenstofffasern, den Zusatz von Stabilisatoren und durch eine Erhöhung des Polymerisationsgrades erzielt werden. Die im Bereich der Hochleistungskunststoffe vorhandene Dauergebrauchstemperatur von mindestens 130 °C wird jedoch erst durch das Ersetzen von aliphatischen durch aromatische Einheiten erreicht. Der Begriff Dauergebrauchstemperatur bezeichnet hierbei die maximale Temperatur, bei der der jeweilige Kunststoff in heißer Luft nach 20.000 Stunden Lagerung höchstens 50 % seiner Ausgangseigenschaften verloren hat. Ergänzend wird in dieser Hinsicht auf die DIN IEC 216 sowie die DIN EN 60216 verwiesen.

Eine höhere Dauergebrauchstemperatur lässt sich durch einen vollständigen Verzicht auf aliphatische Elemente und eine enge Verknüpfung von Aromaten durch funktionelle Gruppen wie Ether-, Sulphon- oder Imid-Gruppen erreichen, so dass Dauergebrauchstemperaturen von mindestens 200 °C bis mindestens 260 °C erzielbar sind. Daher weist der thermoplastische Kunststoff in einer bevorzugten Ausführungsform eine Dauergebrauchstemperatur von mindestens 150 °C, vorzugsweise mindestens 170 °C und besonders bevorzugt von mindestens 190 °C auf. Zusätzlich oder alternativ bevorzugt ist der thermoplastische Kunststoff aus der Gruppe der amorphen Kunststoffe ausgewählt. Amorph ist hierbei generell die Bezeichnung für den Zustand eines festen Stoffs, in dem die Bausteine, also Atome, Ionen oder Moleküle, keine periodische Anordnung über einen größeren Bereich, die sogenannte Fernordnung, aufweisen. Die amorphen thermoplastischen Kunststoffe sind in der Ausgangsform transparent. Zusätzlich ist ein Bauteil aus amorphem thermoplastischem Kunststoff hart im Vergleich zu teilkristallinen thermoplastischen Hochleistungskunststoffen. Ein Nachteil im Vergleich zu den teilkristallinen Kunststoffen ist allerdings, dass er eine geringere Chemikalienbeständigkeit aufweist.

Der Einklebehalter 1 wird mittels Klebstoff stoffschlüssig in der Bauteilöffnung 3 befestigt. Aufgrund der unten näher beschriebenen Konstruktion des Einklebehalters 1 ist es erforderlich, den Funktionseinsatz 40 in dem Einklebehalter 1 anzuordnen, bevor der Einklebehalter 1 in die Bauteilöffnung 3 eingeklebt wird. Nach dem Einkleben des Einklebehalters 1 mit dem Funktionseinsatz 40 in die Bauteilöffnung 3 ist es nicht mehr möglich, den Funktionseinsatz 40 aus dem Einklebehalter 1 zu entfernen oder durch einen anderen Funktionseinsatz 40 auszutauschen. Entsprechend zeigt Figur 2 eine schematische Darstellung des Einklebehalters 1 mit Funktionseinsatz 40, der in der Bauteilöffnung 3 des Bauteils B befestigt ist.

Der Einklebehalter 1 ist mehrteilig und buchsenartig ausgebildet. Die Bezeichnung buchsenartig hat den Hintergrund, dass in dem Einklebehalter 1 der Funktionseinsatz 40 anordenbar und haltbar ist.

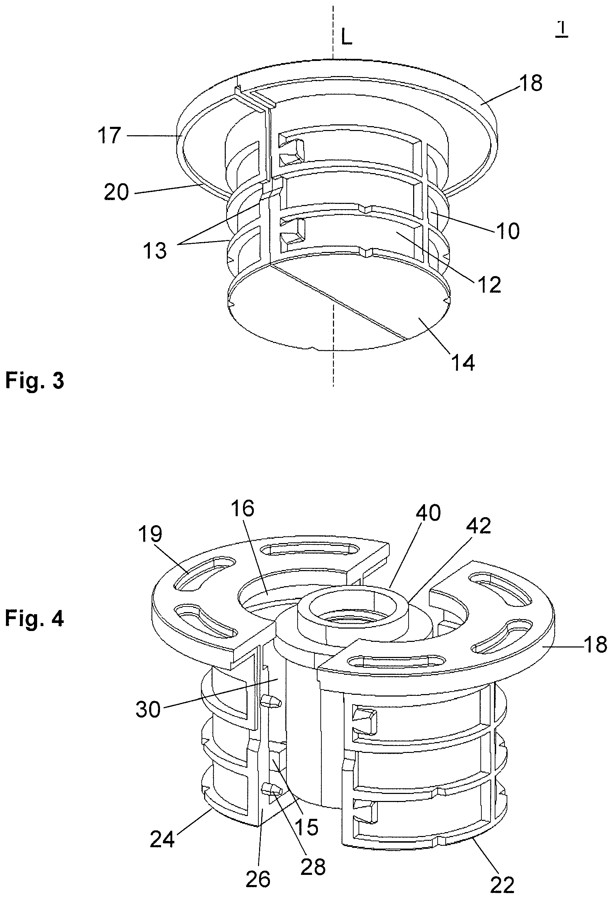

Anhand der Figuren 3 und 4 wird die bevorzugte Konstruktion des Einklebehalters 1 näher erläutert. Der Einklebehalter 1 weist einen topfartigen Aufnahmekörper 10 auf. Dieser ist teilweise hohlzylindrisch ausgebildet, um den Funktionseinsatz 40 aufzunehmen und permanent zu halten.

Der sich entlang der Längsachse L erstreckende Aufnahmekörper 10 für den Funktionseinsatz 40 hat eine umlaufende Mantelfläche 12, eine geschlossene axiale Stirnseite 14 und eine offene axiale Stirnseite 16.

Die offene Stirnseite 16 umgibt vorzugsweise ein umfänglich umlaufender Befestigungskragen 18. Gemäß der gezeigten bevorzugten Ausgestaltung ist der Befestigungskragen 18 geschlossen umfänglich umlaufend ausgebildet. Es ist ebenfalls bevorzugt, den Befestigungskragen 18 mit Durchbrüchen in axialer Richtung zu versehen (nicht gezeigt).

Der Befestigungskragen 18 weist vorzugsweise an seiner bauteilabgewandten Seite eine Mehrzahl gleichmäßig verteilter Vertiefungen 19 auf. Innerhalb der Vertiefungen 19 ist die Dicke des Befestigungskragens 18 in Richtung der Längsachse L so weit reduziert, dass ein Boden der Vertiefung 19 mit Licht durchstrahlbar ist. Die Vertiefungen 19 dienen somit als Durchstrahlfenster, um einen an der bauteilzugewandten Seite des Befestigungskragens 18 befindlichen Klebstoff zu aktivieren und/oder auszuhärten.

Gleichermaßen ist es bevorzugt, den Befestigungskragen 18 ohne die Vertiefungen 19 vorzusehen.

An der bauteilzugewandten Seite des Befestigungskragens 18 ist vorzugsweise ein umfänglich umlaufender Steg 17 vorgesehen, der in axialer Richtung vorsteht. Der Steg 17 begrenzt eine Rinne an der bauteilzugewandten Seite des Befestigungskragens 18, in der Klebstoff aufgenommen werden kann. Vorzugsweise vermeidet der Steg 17 ein Verdrängen von Klebstoff radial auswärts über die radiale Ausdehnung des Befestigungskragens 18 hinaus.

Die Mantelfläche 12 weist eine Mehrzahl radial auswärts vorstehender Stege 13 auf, die parallel zur Längsachse L und senkrecht dazu verlaufen. Die Stege 13 begrenzen Strömungsbahnen für Klebstoff, mit dem der Einklebehalter 1 in der Bauteilöffnung 3 befestigt wird. Vorzugsweise sind die Stege 13 an einer Mehrzahl von Stellen durchbrochen, um Überströmstellen für den Klebstoff zwischen benachbarten Strömungsbahnen zu bilden. Zudem dienen die Stege 13 bevorzugt auch zum Entlüften des Klebebereichs. Das bedeutet, dass Lufteinschlüsse an der Klebestelle beim Einkleben des Einklebehalters 1 in die Sacklochbohrung 3 weitestgehend vermieden werden.

Anhand der Figuren 3 und 4 erkennt man die buchsenartige oder topfartige Gestalt des Aufnahmekörpers 10. Gemäß einer bevorzugten Ausführungsform vorliegender Erfindung ist der Aufnahmekörper 10 durch mindestens einen Axialschnitt 20 zumindest zweiteilig ausgebildet. Gemäß einer Ausführungsform besteht der Aufnahmekörper 10 aus genau zwei Teilen 22, 24, insbesondere aus zwei Hälften 22, 24 oder halbschalen des Aufnahmekörpers 20.

Die beiden Hälften 22, 24 des Aufnahmekörpers 20 weisen jeweils eine Verbindungsfläche 26 auf, die durch den Axialschnitt 20 entstanden ist. Diese Verbindungsflächen 26 sind einander gegenüberliegend angeordnet und liegen aneinander an, wenn der Aufnahmekörper 10 geschlossen ist oder in der Bauteilöffnung 3, vorzugsweise eine Sacklochbohrung, befestigt ist. Eine Flächennormale der Verbindungsfläche 26 ist vorzugsweise senkrecht zur Längsachse L ausgerichtet.

Um die beiden Hälften 22, 24 verlässlich und ausgerichtet einander gegenüberliegend anzuordnen, weisen die Verbindungsflächen 26 vorzugsweise Positionierhilfen 28 und/oder Verbindungshilfen auf. Die Positionier- und/oder Verbindungshilfe 28 ist gemäß einer Ausgestaltung ein Stift, der in eine passende Öffnung eingreift. Es ist ebenfalls bevorzugt, an dieser Stelle eine Schnapp- oder Rastverbindung vorzusehen.

Vorzugsweise sind die beiden Hälften 22, 24 baugleich ausgebildet, was den Herstellungsprozess und den späteren Zusammenbau effektiv gestaltet.

Gemäß einer weiteren bevorzugten Ausgestaltung sind die beiden Hälften 22, 24 über ein Filmscharnier bewegbar miteinander verbunden. Bewegbar bedeutet, dass sich der Aufnahmekörper 10 aus Figur 3 im Gegensatz zu Figur 4 aufklappen lässt, um den Funktionseinsatz 40 einzusetzen. Gemäß unterschiedlicher bevorzugter Ausgestaltungen vorliegender Erfindung ist das Filmscharnier (nicht gezeigt) radial außen am Befestigungskragen 18 oder am Boden des Aufnahmekörpers 10 angeordnet. Die jeweilige Positionierung des Filmscharniers würde ein Öffnen des Aufnahmekörpers 10 zum Aufnehmen/Einsetzen des Funktionseinsatzes 40 und nachfolgenden Schließen des Aufnahmekörpers 10 gewährleisten. Vorzugsweise ist das Filmscharnier in der Konstruktion des unten beschriebenen Blockierstegs 15 realisiert.

Es ist ebenfalls bevorzugt, den Aufnahmekörper 10 durch Axialschnitte 20 in mehr als zwei Teile 22, 24 zu unterteilen, um den Funktionseinsatz 40 einsetzen zu können.

Der Aufnahmekörper 10 weist eine Funktionsinnenseite 30 auf, die der umgebenden Mantelfläche 12 gegenüber angeordnet ist. Die Funktionsinnenseite 30 dient der Befestigung und dem Halten des Funktionseinsatzes 40 im Inneren des Aufnahmekörpers 10. Gemäß einer ersten bevorzugten Ausgestaltung (siehe Figur 4) weist die Funktionsinnenseite 30 mindestens eine umlaufende Nut 32 oder Vertiefung auf, in der ein passend ausgebildeter Radialkragen 42 des Funktionseinsatzes 40 aufnehmbar ist. Vorzugsweise ist die Nut 32 vollständig umlaufend ausgebildet. Es ist ebenfalls bevorzugt, die Nut 32 an mindestens einer Position durch einen aus der Funktionsinnenseite 30 vorstehenden Blockiersteg (nicht gezeigt) zu unterbrechen. Der Blockiersteg greift in eine passende Ausnehmung (nicht gezeigt) im Radialkragen 42 ein, um auf diese Weise eine Verdrehsicherung des Funktionseinsatzes 40 im Aufnahmekörper 10 bereitzustellen.

Die umlaufende Nut 32 und der dort eingreifende Radialkragen 42 sind vorzugsweise in ihren Dimensionen aufeinander abgestimmt. Gemäß einer bevorzugten Ausgestaltung ist der Radialkragen 42 in seiner Größe genau passend zur Nut 32 vorgesehen, so dass der Radialkragen 42 und damit der Funktionseinsatz 40 fest in der Nut 32 gehalten wird. Aufgrund dieser Verbindung ist weder ein radialer noch ein axialer Versatz des Funktionseinsatzes 40 innerhalb des Aufnahmekörpers 10 möglich.

Gemäß einer anderen bevorzugten Ausgestaltung sind die Nut 32 und der Radialkragen 42 derart ausgebildet, dass der Funktionseinsatz 40 an einer festen axialen Position im Aufnahmekörper 10 gehalten wird. Gleichzeitig ist es aber ebenfalls möglich, dass sich der Radialkragen 42 und damit der Funktionseinsatz 40 radial in der Nut 32 versetzen kann. Auf diese Weise ist eine schwimmende Lagerung des Funktionseinsatzes 40 im Aufnahmekörper 10 realisiert.

Es versteht sich, dass der Radialkragen auch an der Funktionsinnenseite 30 angeordnet sein kann. In diesem Fall weist die Außenseite des Funktionseinsatzes 40 die passend ausgebildete Nut zum Aufnehmen des Radialkragens auf.

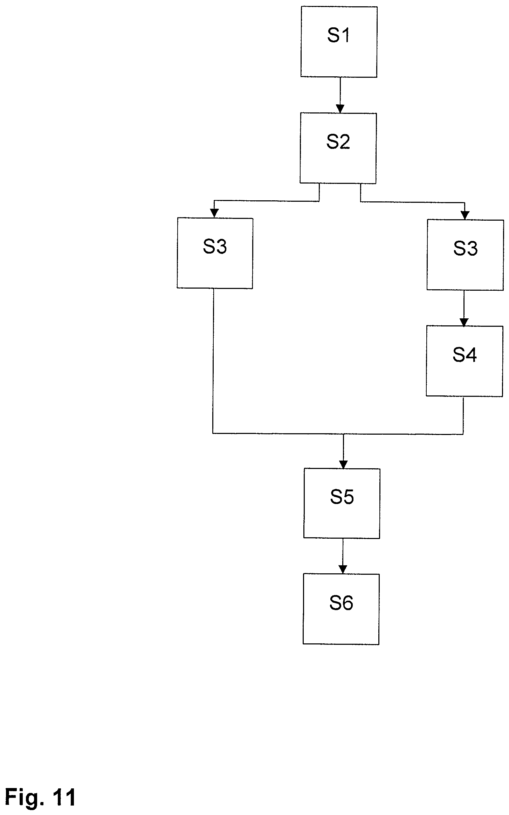

An einer Innenseite der geschlossenen Stirnseite 14 ist bevorzugt ein Blockiersteg 15 vorgesehen. Der Blockiersteg 15 steht in axialer Richtung aus der Innenseite der geschlossenen Stirnseite 14 vor und ist vorzugsweise außermittig bezogen auf die Stirnseite 14 angeordnet. Ebenfalls bevorzugt verläuft der Blockiersteg 15 in radialer Richtung, wie es in Figur 4 beispielgebend dargestellt ist. Der Blockiersteg 15 greift in eine dazu passende Vertiefung 44 an dem Funktionseinsatz 40 ein, um eine Relativdrehung zwischen dem Aufnahmekörper 10 und dem Funktionseinsatz 40 zu verhindern. Falls der Funktionseinsatz 40 schwimmend im Aufnahmekörper 10 gehalten ist, greift der Blockiersteg 15 mit Spiel in die Vertiefung 44 ein. Entsprechend ist die Vertiefung 44 breiter als der Blockiersteg ausgebildet, wie in Fig. 11 gezeigt ist. Im Falle einer festen Anordnung des Funktionseinsatzes 40 im Aufnahmekörper 10 besteht bevorzugt kein Spiel zwischen Blockiersteg 15 und Vertiefung 44.

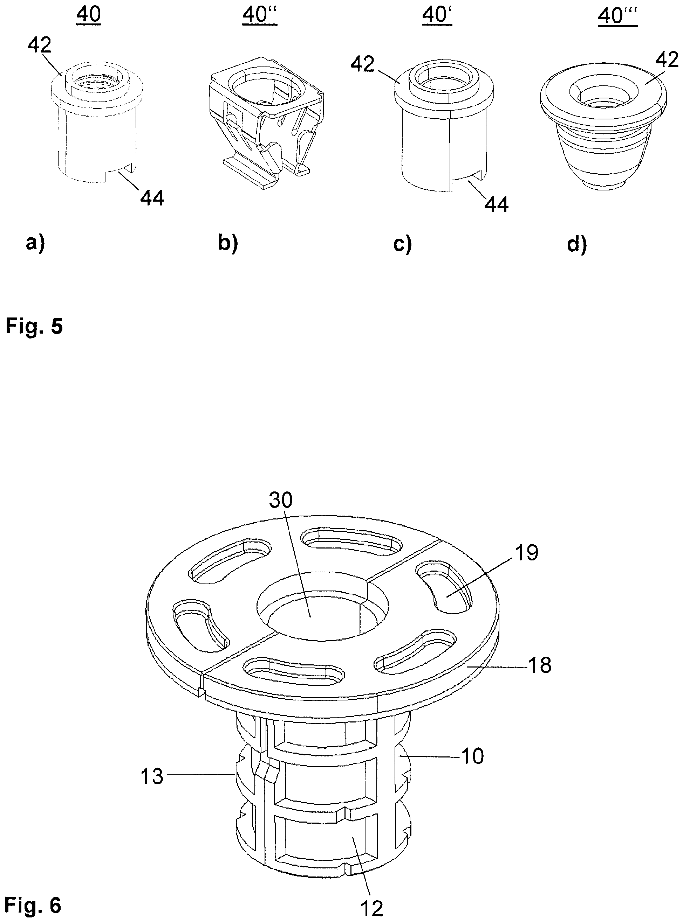

Figur 5 zeigt eine bevorzugte Auswahl von Funktionseinsätzen 40, die schwimmend im Aufnahmekörper 10 angeordnet und gehalten werden können. Der Funktionseinsatz 40 in Figur 5A weist ein Innengewinde auf, welches bevorzugt mit einem gewindeverstärkenden Drahtgewindeeinsatz ausgestattet ist.

Der Funktionseinsatz 40" in Figur 5B ist eine Federklammer für einen Schnellverschluss oder eine Rastverbindung.

Der Funktionseinsatz 40' in Figur 5C hat eine ebene Innenwand, um dort eine gewindefurchende Schraube einzudrehen.

Der Funktionseinsatz 40‴ in Figur 5D ist eine Kugelpfanne, also eine Kupplungsaufnahme, um vorzugsweise einen Kugelbolzen darin lösbar zu halten.

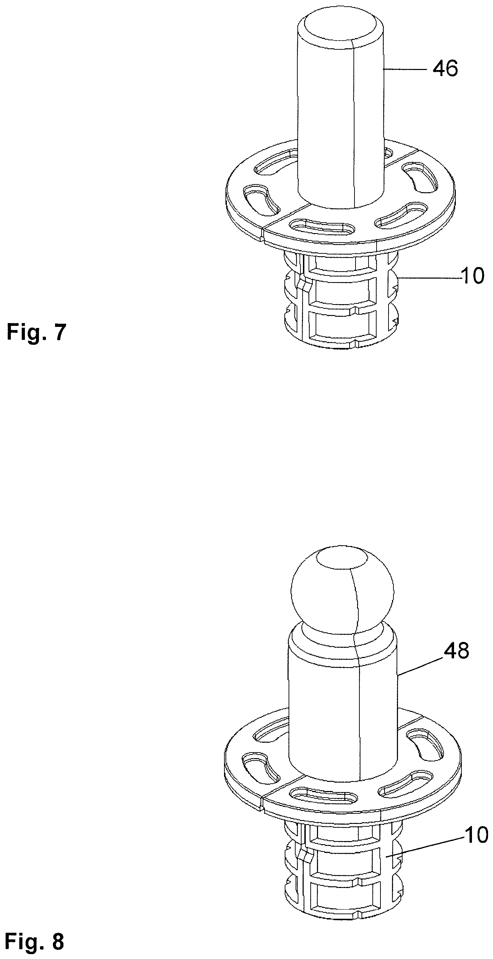

In den Figuren 6 bis 8 sind bevorzugte Ausführungsformen vorliegender Erfindung gezeigt, in denen der Funktionseinsatz 40 fest, also ohne schwimmende Lagerung, im Aufnahmekörper 10 gehalten ist. Der Funktionseinsatz 40 in Figur 6 weist dazu vorzugsweise ein Innengewinde mit oder ohne verstärkenden Drahtgewindeeinsatz auf. In Figur 7 weist der Funktionsansatz 40 neben seiner bevorzugten und nicht zu erkennenden Haltestruktur aus Radialkragen 42 und Vertiefung 44 im Aufnahmekörper 10 einen über den Aufnahmekörper 10 hinausragenden Gewindebolzen 46 auf. Anstelle des Gewindebolzens 46 zeigt die bevorzugte Ausführungsform in Figur 8 einen Kupplungsbolzen 48, vorzugsweise einen Kugelbolzen.

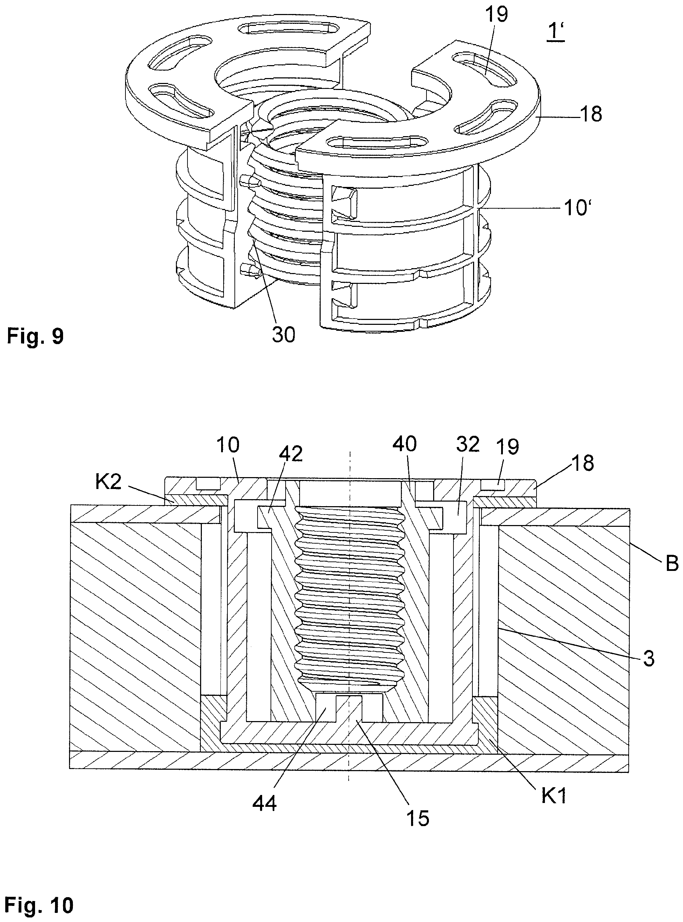

Eine weitere bevorzugte Ausführungsform vorliegender Erfindung zeigt Figur 9. In dem Aufnahmekörper 10 ist in der Funktionsinnenseite 30 ein Gewinde vorgesehen. Als Funktionseinsatz 40ʺʺ ist ebenfalls bevorzugt in dem Gewinde ein Drahtgewindeeinsatz angeordnet. In diesem Zusammenhang hat sich die mehrteilige Konstruktion des Aufnahmekörpers 10 als vorteilhaft erwiesen. Denn dadurch lässt sich der Drahtgewindeeinsatz im geöffneten Zustand des Aufnahmekörpers 10 im Gewinde vorpositionieren (siehe Figur 9) und über das Schließen des Aufnahmekörpers 10 im Gewinde fest anordnen.

Figur 10 zeigt in einer schematischen Schnittansicht den Einklebehalter 1 mit dem Funktionseinsatz 40, der in der Bauteilöffnung 3 klebend befestigt ist.

Bevor dieser Zustand erreicht ist, wird zunächst im Schritt S1 des Installationsverfahrens das Bauteil B mit der Bauteilöffnung 3, vorzugsweise eine Sacklochbohrung, bereitgestellt.

Nachfolgend oder bereits vor dem Installationsverfahren wird der Aufnahmekörper 10 geöffnet und ein Funktionseinsatz 40 gewünschter Ausgestaltung im Aufnahmekörper 10 angeordnet. Nachfolgend wird der Aufnahmekörper 10 wieder geschlossen, um den Funktionseinsatz 40 darin untrennbar zu halten (Schritt S2). Das Öffnen und Schließen des Aufnahmekörpers 10 erfolgt über das oben beschriebene bevorzugte Filmscharnier. Alternativ dazu wird bevorzugt der Aufnahmekörper 10 in zwei Hälften oder andere Einzelteile zerlegt und nach positioniertem Funktionseinsatz wieder zusammengesetzt.

Gemäß einer ersten Installationsvariante wird eine erste Klebstoffmenge K1 am Boden der Sacklochbohrung 3 appliziert (Schritt S3).

Nachfolgend wird im Schritt S5 der Einklebehalter 1 mit dem Funktionseinsatz 40 in die Sacklochbohrung 3 eingesetzt. Da der Einklebehalter 1 in die erste Klebstoffmenge K1 eintaucht, wird dadurch die erste Klebstoffmenge K1 in den Spalt zwischen der Innenwand der Sacklochbohrung 3 und der Mantelfläche 12 des Einklebehalters 1 verdrängt. Ist die Menge des ersten Klebstoffs K1 ausreichend groß, erfolgt eine Verteilung der ersten Klebstoffmenge K1 bis unter den Befestigungskragen 18. Die erste Klebstoffmenge K1 wird danach im Schritt S6 ausgehärtet.

Das Aushärten der ersten Klebstoffmenge K1 erfolgt vorzugsweise mittels Wärme oder über Zeitablauf einer bekannten Aushärtezeit. Zudem ist es bevorzugt, einen durch Licht aktivierbaren und/oder aushärtbaren Klebstoff einzusetzen. In diesem Fall wird über die bevorzugten Durchstrahlfenster 19 oder direkt durch den Befestigungskragen 18 Licht auf die erste Klebstoffmenge K1 unter dem Befestigungskragen 18 eingestrahlt und dessen Aktivierung und/oder Aushärtung gestartet.

Gemäß einer weiteren bevorzugten Installationsroute wird die erste Klebstoffmenge K1 am Boden der Sacklochbohrung 3 appliziert (Schritt S3). Vorzugsweise ist die erste Klebstoffmenge K1 in ihrem Volumen so weit begrenzt, dass sie maximal nur den Spalt zwischen der Innenwand der Sacklochbohrung 3 und der Mantelfläche 12 benetzt. Entsprechend erreicht die erste Klebstoffmenge K1 nicht die Bauteiloberfläche unterhalb des Befestigungskragens 18.

In einem weiteren bevorzugten Schritt S4 wird eine zweite Klebstoffmenge K2 auf den Oberflächenbereich angrenzend an die Öffnung der Sacklochbohrung 3 appliziert, der durch den Befestigungskragen 18 abgedeckt sein wird.

Nachfolgend wird der Einklebehalter 1 in die Sacklochbohrung 3 eingesetzt (Schritt S5) und es werden die erste K1 und die zweite Klebstoffmenge K2 ausgehärtet (Schritt S6).

Gemäß einer bevorzugten Ausgestaltung des Installationsverfahrens sind die Klebstofftypen der ersten K1 und der zweiten Klebstoffmenge K2 verschieden. So umfasst die zweite Klebstoffmenge K2 einen durch Licht aktivierbaren und/oder aushärtbaren Klebstoff. Entsprechend wird nach dem Einsetzen des Einklebehalters 1 in die Sacklochbohrung 3 Licht klebstoffspezifischer Wellenlänge über die Durchstrahlfenster 19 in dem Befestigungskragen 18 auf die zweite Klebstoffmenge K2 unter dem Befestigungskragen 18 eingestrahlt. Dadurch wird die zweite Klebstoffmenge K2 aktiviert oder aktiviert und ausgehärtet. Dies eröffnet die bevorzugte Möglichkeit, den Einklebehalter 1 in der Sacklochbohrung 3 über den Befestigungskragen 18 und die lichtaktivierte zweite Klebstoffmenge K2 vorzufixieren.

Vorzugsweise umfasst die erste Klebstoffmenge K1 einen durch Wärme aushärtenden Klebstoff. Entsprechend wird der Sacklochbohrung 3 mit vorfixiertem Einklebehalter 1 gezielt eine aushärtende Wärmemenge zugeführt, um die erste Klebstoffmenge K1 auszuhärten.

Ebenfalls bevorzugt wird als Klebstoff der ersten Klebstoffmenge K1 ein über Zeitablauf selbstständig aushärtender Klebstoff genutzt. Während der Aushärtephase der ersten Klebstoffmenge K1 sorgt die oben beschriebene Vorfixierung mit Hilfe der zweiten Klebstoffmenge K2 für eine verlässliche Positionierung und den nötigen Halt des Einklebehalters 1 mit Funktionseinsatz 40 in der Bauteilöffnung 3.

Gemäß der in Figur 10 gezeigten bevorzugten Ausführungsform vorliegender Erfindung wird der Einklebehalter 1 in die Sacklochbohrung 3 einer Sandwichplatte B eingeklebt. In der Sandwichplatte B bekannter Konstruktion ist beispielsweise eine Wabenplatte oder ein Wabenkern zwischen der oberen und unteren Decklage angeordnet. Die Decklagen sind stabiler als die Wabenplatte und mit der Wabenplatte verklebt, um eine stabile und mechanisch belastbare Plattenkonstruktion zu erzielen.

Der eingeklebte Einklebehalter 1 in der Sandwichplatte B ist bevorzugt mit beiden Decklagen verklebt. Auf diese Weise wird eine zusätzliche strukturelle Kopplung zwischen den Decklagen hergestellt. Damit wird der Einklebehalter 1 über die strukturell stabilsten Bestandteile der Sandwichplatte B gehalten, was die nötige Stabilität erzeugt. Zudem können über den Einklebehalter 1 mechanische Kräfte auf beide Decklagen abgeleitet werden, wodurch die Sandwichplatte stabilisiert wird.

Anstelle einer Sandwichplatte wird der Einklebehalter 1 bevorzugt in gleicher Weise in eine Sacklochbohrung 3 eines Bauteils B aus Kunststoff oder Holz oder Metall oder ähnlichem als Vollmaterial oder mehrlagige Struktur eingeklebt. Dies erfolgt mit einem oder zwei Klebstoffsorten und/oder -mengen, wie es oben beschrieben worden ist.

Zum Herstellen des erfindungsgemäßen Einklebehalters gemäß seinen bevorzugten Ausgestaltungen wird auf bekannte Herstellungsverfahren, wie den Spritzguss oder additive Herstellungsverfahren, wie den 3D-Druck oder das Lasersintern, zurückgegriffen. Beispielgebend werden anhand des bevorzugten Spritzgussverfahrens die Herstellungsschritte zusammengefasst. Zunächst werden eine erste Spritzgussform, die komplementär zu der Form des Einklebehalters 1, insbesondere zu dem Aufnahmekörper 10, ausgebildet ist, und bevorzugt eine zweite Spritzgussform bereitgestellt, die komplementär zu der Form des Funktionseinsatzes 40 ausgebildet ist. Nachfolgend findet das Spritzgießen des Aufnahmekörpers 10 und bevorzugt des Funktionseinsatzes 40 statt. Gemäß einer Ausgestaltung des Herstellungsverfahrens werden der Aufnahmekörper 10 und der Funktionseinsatz 40 aus einem Hochleistungskunststoff (siehe oben) produziert. Es ist aber ebenfalls bevorzugt, andere aufgrund ihrer Eigenschaften geeignete Kunststoffe einzusetzen. Nach dem Spritzgießen werden der Aufnahmekörper und bevorzugt der Funktionseinsatz entformt.

### Bezugszeichenliste

- 1: Einklebehalter
- 3: Bauteilöffnung
- 10: topfartiger Aufnahmekörper
- 12: Mantelfläche
- 13: Stege der Mantelfläche
- 14: geschlossene axiale Stirnseite
- 15: Blockiersteg
- 16: offene axiale Stirnseite
- 17: umlaufender Steg
- 18: Befestigungskragen
- 19: Vertiefung oder Durchstrahlfenster im Befestigungskragen 18
- 20: Axialschnitt
- 22, 24: Hälften des Aufnahmekörpers 10
- 26: Verbindungsfläche
- 28: Positionier- und/oder Verbindungshilfe der Hälften 22, 24
- 30: Funktionsinnenseite
- 33: umlaufende Nut
- 40: Funktionseinsatz
- 42: Radialkragen
- 44: Vertiefung für Blockiersteg 15
- 46: Gewindebolzen
- 48: Kugelbolzen
- 50: Drahtgewindeeinsatz
- B: Bauteil
- L: Längsachse des Eigenkleberhalters

## Patentansprüche

1. Ein mehrteilig buchsenartiger Einklebehalter (1), der in einer Bauteilöffnung (3) klebend befestigbar und aufgrund einer Axialteilung zumindest zweiteilig und dadurch in Radialrichtung öffnend ausgebildet ist, so dass
nur vor einem Einkleben des Einklebehalters (1) in einer Bauteilöffnung (3) ein Funktionseinsatz (40) in dem Einklebehalter (1) anordenbar ist, um
nach dem Einkleben des Einklebehalters (1) in der Bauteilöffnung (3)
in dem Einklebehalter (1) untrennbar gehalten zu sein, wobei der Einklebehalter (1) einen hohlzylindrischen Aufnahmekörper (10) mit einer umlaufenden Mantelfläche (12), einer geschlossenen axialen Stirnseite (14) und einer offenen axialen Stirnseite (16) mit einer Funktionsöffnung (16) aufweist, die gegenüber der geschlossenen axialen Stirnseite (14) vorgesehen ist, **dadurch gekennzeichnet, dass** angrenzend an die Funktionsöffnung (16) der offenen Stirnseite (14) ein umfänglich umlaufender Befestigungskragen (18) angeordnet ist, der sich radial in Bezug auf die Mantelfläche (12) des Aufnahmekörpers (10) erstreckt.

2. Der Einklebehalter (1) gemäß Patentanspruch 1, dessen Aufnahmekörper (10) aufgrund mindestens eines Axialschnitts (20) in Längsrichtung in zwei relativ zueinander bewegbare, insbesondere aufklappbar versetzbare, Teile (22, 24) geteilt ist, die vollständig oder teilweise voneinander lösbar sind.

3. Der Einklebehalter (1) gemäß Patentanspruch 2, in dem entlang des Axialschnitts Verbindungsflächen (26) der Teile (22, 24) vorgesehen sind, die einander gegenüber liegen und Positionierhilfen und/oder Verbindungshilfen (28) der Teile (22, 24) aufweisen.

4. Der Einklebehalter (1) gemäß Patentanspruch 2 oder 3, in dem aneinander angrenzende Teile (22, 24) über ein Filmscharnier und/oder eine Schnappverbindung und/oder eine Steckverbindung bewegbar miteinander verbindbar sind.

5. Der Einklebehalter (1) gemäß Patentanspruch 1, in dem die Mantelfläche (12) eine strömungsleitende Außenseite angepasst an eine Leitung eines strömenden Klebstoffs und eine Funktionsinnenseite (30) aufweist, mit der der Funktionseinsatz (40) formschlüssig in dem geschlossenen Aufnahmekörper (10) haltbar ist.

6. Der Einklebehalter (1) gemäß mindestens einem der vorhergehenden Patentansprüche, in dem der Befestigungskragen (18) eine Mehrzahl von Durchstrahlabschnitten (19) aufweist, in denen eine Stärke des Befestigungskragens (18) in Längsrichtung des Einklebehalters (1) reduziert ist, um den Befestigungskragen (18) in den Durchstrahlabschnitten mit Licht durchstrahlbar vorzusehen.

7. Der Einklebehalter (1) gemäß Patentanspruch 5, dessen Funktionsinnenseite (30) eine umlaufende Haltenut (32) aufweist, die in einer Breite und in einer Tiefe an einen umlaufenden Kragen (42) des Funktionseinsatzes (40) angepasst ist, um diesen fest oder schwimmend innerhalb des Aufnahmehohlraums zu halten.

8. Der Einklebehalter (1) gemäß mindestens einem der vorhergehenden Patentansprüche 1 bis 7, der innerhalb des Aufnahmekörpers (10) an der geschlossenen axialen Stirnseite (14) einen Blockiersteg (15) aufweist, der in einer Blockiernut (44) des Funktionseinsatzes (40) aufnehmbar ist, so dass eine Relativdrehung zwischen dem Funktionseinsatz (40) und dem Aufnahmekörper (10) verhindert ist.

9. Der Einklebehalter (1) gemäß mindestens einem der vorhergehenden Patentansprüche, in dem der Funktionseinsatz (40)
a) eine einseitig geschlossene Hülse (40; 40') mit umlaufendem Kragen (42) und einem Innengewinde oder einer planen Innenwand
b) eine Federklammer (40") für einen Schnellverschluss,
c) eine Kugelpfanne (40‴) mit einem umlaufenden Kragen (42) oder
d) einen vorstehenden Gewindezapfen (46) oder Kugelbolzen (48) aufweist.

10. Der Einklebehalter (1) gemäß Patentanspruch 1, in dem der hohlzylindrische Aufnahmekörper (10) an einer umlaufenden Innenwand (30) ein Innengewinde aufweist, in dem als Funktionseinsatz ein Drahtgewinde (50) angeordnet ist.

11. Der Einklebehalter (1) gemäß mindestens einem der vorhergehenden Patentansprüche, in dem der Einklebehalter (1) und/oder der Funktionseinsatz (40) aus einem thermoplastischen Kunststoff mit einer Dauergebrauchstemperatur von mindestens 130 °C bestehen.

12. Ein Bauteil (B) mit einer Bauteilöffnung (3), vorzugsweise eine Sacklochbohrung, in der ein Einklebehalter (1) gemäß einem der vorhergehenden Patentansprüche eingeklebt ist.

13. Ein Installationsverfahren eines Einklebehalters (1) gemäß mindestens einem der vorhergehenden Patentansprüche 1 bis 11 in einem Bauteil (B), welches die folgenden Schritte aufweist:
a) Bereitstellen (S1) eines Bauteils (B) mit einer Sacklochbohrung (3), in die der Einklebehalter (1) aufnehmbar ist,
b) Anordnen (S2) eines Funktionseinsatzes in dem Einklebehalter (1), so dass der Funktionseinsatz (40; 40'; 40"; 40‴;50) unlösbar in dem Einklebehalter (1) gehalten ist, und danach
c) Einkleben des Einklebehalters (1) in der Sacklochbohrung (B) durch
d) Applizieren (S3; S4) einer ersten Klebstoffmenge (K1) an einem Boden der Sacklochbohrung und einer zweiten Klebstoffmenge (K2) zwischen einem umfänglich umlaufenden stirnseitigen Befestigungskragen (18) des Einklebehalters (1) und dem Bauteil (B).
e) Einsetzen (S5) des Einklebehalters (1) mit Funktionseinsatz (40; 40'; 40"; 40‴;50) in die Sacklochbohrung (B) mit der ersten und zweiten Klebstoffmenge (K1; K2) und
f) Aushärten (S6) des Klebstoffs (K1; K2).

14. Das Installationsverfahren gemäß Patentanspruch 13, in dem der Einklebehalter (1) mit zwei verschiedenen Klebstoffen befestigt wird, die unterschiedlichen Aushärteprinzipien gehorchen.

15. Ein Herstellungsverfahren für einen Einklebehalter (1) gemäß einem der vorhergehenden Patentansprüche 1 bis 11, das die folgenden Schritte aufweist:
a) Bereitstellen einer ersten Spritzgussform, die komplementär zu der Form des Einklebehalters (1) ausgebildet ist,
b) Spritzgießen des Einklebehalters (1) in der ersten Spritzgussform und des Funktionseinsatzes (40; 40'; 40‴) in einer zweiten Spritzgussform und
c) Entformen des Einklebehalters (1).

16. Herstellungsverfahren gemäß Patentanspruch 15, das den weiteren Schritt aufweist:
d) Bereitstellen der zweiten Spritzgussform, die komplementär zu der Form des Funktionseinsatzes (40; 40'; 40‴) ausgebildet ist,
e) Spritzgießen des Funktionseinsatzes (40; 40'; 40‴) in der zweiten Spritzgussform und
f) Entformen des Funktionseinsatzes (40; 40'; 40‴).

17. Herstellungsverfahren gemäß Patentanspruch 15, das den weiteren Schritt aufweist:
g) Bereitstellen eines vorgefertigten Funktionseinsatzes (40").

18. Herstellungsverfahren gemäß Patentanspruch 15 oder 16, in dem der Einklebehalter (1) und/oder der Funktionseinsatz (40; 40'; 40‴) aus einem thermoplastischen Kunststoff mit einer Dauergebrauchstemperatur von mindestens 130 °C hergestellt werden.

## Claims

1. A multipart socket-like glue-in retainer (1) which is fastenable in a component opening (3) in an adhesive manner and which is configured in at least two parts due to an axial separation and thus in an opening manner in radial direction, so that
only before a gluing-in of the glue-in retainer (1) in a component opening (3), a functional insert (40) is arrangeable in the glue-in retainer (1) so that
after the gluing-in of the glue-in retainer (1) in the component opening (3), it is retained inseparably in the glue-in retainer (1), wherein
the glue-in retainer (1) comprises a hollow-cylindrical receiving body (10) with a circumferential lateral area (12), a closed axial face side (14) and an open axial face side (16) with a functional opening (16) that is provided opposite to the closed axial face side (14), **characterized in that** adjacent to the functional opening (16) of the open face side (14), a circumferentially surrounding fastening collar (18) is arranged, extending radially with respect to the lateral area (12) of the receiving body (10).

2. The glue-in retainer (1) according to claim 1, the receiving body (10) of which is separated due to at least one axial cut (20) in longitudinal direction into two parts (22, 24) which are moveable relatively towards each other, in particular, they are offsetable by being opened, wherein the parts (22, 24) are completely or partly releasable from one another.

3. The glue-in retainer (1) according to claim 2, in which connecting surfaces (26) of the parts (22, 24) are provided along the axial cut which are arranged opposite to one another and comprise positioning aids and/or connecting aids (28) of the parts (22, 24).

4. The glue-in retainer (1) according to claim 2 or 3, in which parts (22, 24) which are arranged adjacent one another are movably connectable with one another via a film hinge and/or a snap-connection and/or plug connection.

5. The glue-in retainer (1) according to claim 1, in which the lateral area (12) comprises an outside which leads or guides the flow and is adapted to a guidance of a flowing adhesive and comprises a functional inside (30) with which the functional insert (40) is retainable in the closed receiving body (10) in a form-fit manner.

6. The glue-in retainer (1) according to at least one of the preceding claims, in which the fastening collar (18) includes a number of irradiation sections (19) in which a thickness of the fastening collar (18) in longitudinal direction of the glue-in retainer (1) is reduced in order to provide the fastening collar (18) in the irradiation sections such that it may be shone through with light.

7. The glue-in retainer (1) according to claim 5, the functional inside (30) of which includes a circumferential retaining groove (32) which is adapted to a circumferential collar (42) of the functional insert (40) in terms of a width and a depth in order to hold same firmly or swimmingly/floatingly within the receiving cavity.

8. The glue-in retainer (1) according to at least one of the preceding claims 1 to 7, which comprises a blocking web (15) within the receiving body (10) at the closed axial face side (14), the blocking web (15) being receivable in a blocking groove (44) of the functional insert (40) so that a relative rotation between the functional insert (40) and the receiving body (10) is prevented.

9. The glue-in retainer (1) according to at least one of the preceding claims, in which the functional insert (40)
a) is a sleeve (40; 40') being closed on one side and having a circumferential collar (42) and an inner thread or a planar inner wall,
b) a spring clip (40") for a quick lock,
c) a ball socket (40‴) with a circumferential collar (42) or
d) a projecting thread pin (46) or a ball bolt (48).

10. The glue-in retainer (1) according to claim 1, in which the hollow cylindrical receiving body (10) has an inner thread at a circumferential inner wall (30) in which a wire thread insert (50) is arranged as a functional insert.

11. The glue-in retainer (1) according to at least one of the preceding claims, in which the glue-in retainer (1) and/or the functional insert (40) preferably consist(s) of a thermoplastic plastic material with a permanent usage temperature of at least 130°C.

12. A component (B) with a component opening (3), preferably a blind bore hole, in which a glue-in retainer (1) according to one of the preceding claims is glued in.

13. An installation method of a glue-in retainer (1) according to at least one of the preceding claims 1 to 11 in a component (B), comprising the following steps:
a) providing (S1) a component (B) with a blind bore hole (3) in which the glue-in retainer (1) is receivable,
b) arranging (S2) a functional insert in the glue-in retainer (1) so that the functional insert (40; 40'; 40"; 40‴; 50) is retained inseparably in the glue-in retainer (1), and subsequently
c) gluing-in the glue-in retainer (1) in the blind bore hole (B) by
d) applying (S3; S4) a first adhesive amount (K1) to a bottom of the blind bore hole and a second adhesive amount (K2) between a circumferentially surrounding face-sided fastening collar (18) of the glue-in retainer (1) and the component (B),
e) inserting (S5) of the glue-in retainer (1) with functional insert (40; 40'; 40"; 40‴; 50) into the blind bore hole (B) with the first and the second adhesive amount (K1; K2) and
f) curing (S6) of the adhesive (K1; K2).

14. The installation method according to claim 13, in which the glue-in retainer (1) is fastened with two different adhesives which comply with different curing principles.

15. A manufacturing method for a glue-in retainer (1) according to one of the preceding claims 1 to 11, including the following steps:
a) providing of a first injection mold which is configured complimentary to the form of the glue-in retainer (1),
b) injection-molding of the glue-in retainer (1) in the first injection mold and of the functional insert (40; 40'; 40‴) in a second injection mold and
c) demolding of the glue-in retainer (1).

16. Manufacturing method according to claim 15, including the further step:
d) providing the second injection mold which is configured complimentary to the form of the functional insert (40; 40' : 40‴),
e) injection-molding of the functional insert (40; 40'; 40‴) in the second injection mold and
f) demolding of the functional insert (40; 40'; 40‴).

17. Manufacturing method according to claim 15, including the further step:
g) providing a prefabricated functional insert (40").

18. Manufacturing method according to claim 15 or 16, in which the glue-in retainer (1) and/or the functional insert (40; 40'; 40‴) are manufactured from a thermoplastic plastic material with a permanent usage temperature of at least 130°C.

## Revendications

1. Dispositif de retenue collable (1) du type douille en plusieurs parties, lequel peut être fixé par collage dans une ouverture de composant (3) et lequel est conçu en au moins deux parties en raison d'une division axiale et donc de manière à s'ouvrir dans une direction radiale, de telle façon que
un insert fonctionnel (40) peut être disposé dans le dispositif de retenue collable (1) uniquement avant un collage du dispositif de retenue collable (1) dans une ouverture de composant (3), afin d'être maintenu de façon inséparable dans le dispositif de retenue collable (1) après le collage du dispositif de retenue collable (1) dans l'ouverture de composant (3), dans lequel le dispositif de retenue collable (1) présente un corps de réception cylindrique creux (10) avec une surface d'enveloppe circonférentielle (12), un côté avant axial fermé (14) et un côté avant axial ouvert (16) avec une ouverture fonctionnelle (16), laquelle est prévue en face du côté avant axial fermé (14), **caractérisé en ce qu'**un col de fixation circonférentiel périphérique (18) est disposé de façon adjacente à l'ouverture fonctionnelle (16) du côté avant ouvert (14), lequel s'étend radialement par rapport à la surface d'enveloppe (12) du corps de réception (10).

2. Dispositif de retenue collable (1) selon la revendication 1, dont le corps de réception (10) est divisé dans la direction longitudinale en deux parties (22, 24) mobiles l'une par rapport à l'autre, en particulier déplaçables de façon ouvrable du fait d'au moins une coupe axiale (20), lesquelles peuvent être détachées complètement ou partiellement l'une de l'autre.

3. Dispositif de retenue collable (1) selon la revendication 2, dans lequel des surfaces d'assemblage (26) des parties (22, 24) sont prévues le long de la coupe axiale, lesquelles se situent l'une en face de l'autre et présentent des aides au positionnement et/ou des aides à l'assemblage (28) des parties (22, 24).

4. Dispositif de retenue collable (1) selon la revendication 2 ou 3, dans lequel des parties (22, 24) adjacentes peuvent être reliées entre elles de façon mobile par le biais d'une charnière à film et/ou d'un assemblage à encliquetage et/ou d'un assemblage à enfichage.

5. Dispositif de retenue collable (1) selon la revendication 1, dans lequel la surface d'enveloppe (12) présente un côté extérieur conducteur de fluide adapté à un conduit d'une colle en écoulement et un côté intérieur fonctionnel (30), avec lequel l'insert fonctionnel (40) peut être retenu par complémentarité de forme dans le corps de réception (10) fermé.

6. Dispositif de retenue collable (1) selon l'une au moins des revendications précédentes, dans lequel le col de fixation (18) présente une pluralité de sections de passage de faisceau (19), dans lesquelles une épaisseur du col de fixation (18) est réduite dans la direction longitudinale du dispositif de retenue collable (1), afin de prévoir un passage de lumière à travers le col de fixation (18) dans les sections de passage de faisceau.

7. Dispositif de retenue collable (1) selon la revendication 5, dont le côté intérieur fonctionnel (30) présente une rainure de retenue circonférentielle (32), dont une largeur et une profondeur sont adaptées à un col circonférentiel (42) de l'insert fonctionnel (40), afin de retenir celui-ci fixement ou de façon flottante à l'intérieur de l'espace creux de réception.

8. Dispositif de retenue collable (1) selon l'une au moins des revendications précédentes 1 à 7, lequel présente une nervure de blocage (15) à l'intérieur du corps de réception (10) sur le côté avant axial fermé (14), laquelle peut être reçue dans une rainure de blocage (44) de l'insert fonctionnel (40) de manière à empêcher une rotation relative entre l'insert fonctionnel (40) et le corps de réception (10).

9. Dispositif de retenue collable (1) selon l'une au moins des revendications précédentes, dans lequel l'insert fonctionnel (40) présente
a) une douille fermée unilatéralement (40 ; 40') avec un col circonférentiel (42) et un filetage intérieur ou une paroi intérieure plane
b) une pince à ressort (40") pour une fermeture rapide,
c) un coussinet sphérique (40‴) avec un col circonférentiel (42) ou
d) un tourillon fileté saillant (46) ou un boulon à rotule (48).

10. Dispositif de retenue collable (1) selon la revendication 1, dans lequel le corps de réception cylindrique creux (10) présente un filetage intérieur sur une paroi intérieure circonférentielle (30), dans lequel est disposé un filetage de fil (50) en tant qu'insert fonctionnel.

11. Dispositif de retenue collable (1) selon l'une au moins des revendications précédentes, dans lequel le dispositif de retenue collable (1) et/ou l'insert fonctionnel (40) sont constitués d'une matière thermoplastique à une température d'utilisation permanente d'au moins 130 °C.

12. Composant (B) doté d'une ouverture de composant (3), de préférence un alésage borgne, dans laquelle/lequel est collé un dispositif de retenue collable (1) selon l'une des revendications précédentes.

13. Procédé d'installation d'un dispositif de retenue collable (1) selon l'une au moins des revendications 1 à 11 dans un composant (B), lequel présente les étapes suivantes :
a) mise à disposition (S1) d'un composant (B) avec un alésage borgne (3), dans lequel le dispositif de retenue collable (1) peut être reçu,
b) disposition (S2) d'un insert fonctionnel dans le dispositif de retenue collable (1), de telle façon que l'insert fonctionnel (40 ; 40' ; 40" ; 40"" ; 50) est retenu de façon non détachable dans le dispositif de retenue collable (1), et ensuite
c) collage du dispositif de retenue collable (1) dans l'alésage borgne (B) par
d) application (S3 ; S4) d'une première quantité de colle (K1) sur un fond de l'alésage borgne et d'une deuxième quantité de colle (K2) entre un col de fixation côté avant circonférentiel périphérique (18) du dispositif de retenue collable (1) et le composant (B),
e) insertion (S5) du dispositif de retenue collable (1) avec l'insert fonctionnel (40 ; 40' ; 40" ; 40"" ; 50) dans l'alésage borgne (B) avec la première et la deuxième quantité de colle (K1 ; K2) et
f) durcissement (S6) de la colle (K1 ; K2).

14. Procédé d'installation selon la revendication 13, dans lequel le dispositif de retenue collable (1) est fixé à l'aide de deux colles différentes, lesquelles obéissent à des principes de durcissement distincts.

15. Procédé d'installation pour un dispositif de retenue collable (1) selon l'une des revendications précédentes 1 à 11, lequel présente les étapes suivantes :
a) mise à disposition d'un premier moule d'injection, lequel est réalisé de façon complémentaire à la forme du dispositif de retenue collable (1),
b) moulage par injection du dispositif de retenue collable (1) dans le premier moule d'injection et de l'insert fonctionnel (40 ; 40' ; 40‴) dans un deuxième moule d'injection et
c) démoulage du dispositif de retenue collable (1).

16. Procédé de fabrication selon la revendication 15, lequel présente l'étape supplémentaire suivante :
d) mise à disposition du deuxième moule d'injection, lequel est réalisé de façon complémentaire à la forme de l'insert fonctionnel (40 ; 40' ; 40‴),
e) moulage par injection de l'insert fonctionnel (40 ; 40' ; 40‴) dans le deuxième moule d'injection et
f) démoulage de l'insert fonctionnel (40 ; 40' ; 40‴).

17. Procédé de fabrication selon la revendication 15, lequel présente l'étape supplémentaire suivante :
g) mise à disposition d'un insert fonctionnel (40") préfabriqué.

18. Procédé de fabrication selon la revendication 15 ou 16, dans lequel le dispositif de retenue collable (1) et/ou l'insert fonctionnel (40 ; 40' ; 40‴) sont fabriqués à partir d'une matière thermoplastique à une température d'utilisation permanente d'au moins 130 °C.
